(19) Europäisches Patentamt
European Patent Office
Office européen des brevets

(11) Veröffentlichungsnummer: **0 084 329**
**B1**

# (12) EUROPÄISCHE PATENTSCHRIFT

(45) Veröffentlichungstag der Patentschrift: **02.04.86**

(51) Int. Cl.[4]: **C 07 C 121/48,** C 07 C 120/00

(21) Anmeldenummer: **83100084.9**

(22) Anmeldetag: **07.01.83**

## (54) Verfahren zur Herstellung von 1,4-Bis-(dicyanomethylen)-cyclohexan.

(30) Priorität: **20.01.82 DE 3201484**

(43) Veröffentlichungstag der Anmeldung:
**27.07.83 Patentblatt 83/30**

(45) Bekanntmachung des Hinweises auf die Patenterteilung:
**02.04.86 Patentblatt 86/14**

(84) Benannte Vertragsstaaten:
**CH DE FR GB IT LI**

(56) Entgegenhaltungen:
**GB - A - 1 291 437**
**US - A - 4 229 364**

**Int. Journal of Methods in Synthetic Organic Chemistry, Nr. 3, März 1978, Seiten 178-183**
**Fieser-Fieser Reagents for Organic Synthesis (1967), Seiten 16,178**

(73) Patentinhaber: **BAYER AG, Konzernverwaltung RP Patentabteilung, D-5090 Leverkusen 1 Bayerwerk (DE)**

(72) Erfinder: **Jonas, Friedrich, Dr., Krugenofen 15, D-5100 Aachen (DE)**
Erfinder: **Hocker, Jürgen, Dr., Eichenweg 6, D-5060 Bergisch-Gladbach 2 (DE)**

EP 0 084 329 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft ein neues Verfahren zur Herstellung von 1,4-Bis-(dicyanomethylen)-cyclohexan.

1,4-Bis-(dicyanomethylen)-cyclohexan ist ein bekanntes und technisch notwendiges Zwischenprodukt für die Herstellung von 7,7,8,8-Tetracyanochinodimethan (TCNQ). Dieses hat die Fähigkeit als Elektronenakzeptor mit Elektronendonatoren Charge-Transfer-Komplexe von hoher elektrischer Leitfähigkeit zu bilden und daher wachsende technische Bedeutung (s. J. Am. Chem. Soc. 84, 3374 [1962]).

Gegenstand der Erfindung ist ein Verfahren zur direkten Herstellung von 1,4-Bis-(dicyanomethylen)--cyclohexan, das dadurch gekennzeichnet ist, dass man einen Cyclohexan-1,4-dion-2,5-dicarbonsäure--($C_1$-$C_6$)-alkylester mit Wasser bei einem Druck von 1 bis 200 bar und einer Temperatur von 80 bis 250°C behandelt und die erhaltene wässrige Lösung mit Malonsäuredinitril, gegebenenfalls in Anwesenheit eines Katalysators, bei Temperaturen von 0 bis 100°C umsetzt.

Die Reaktion verläuft im allgemeinen mit einer Gesamtausbeute von mehr als 90% der Theorie.

Jede der beiden Stufen dieses Verfahrens für sich allein ist aus Fieser-Fieser, Reagents for Organic Synthesis, John Wiley and Sons, New York, 1967, Seite 16 bzw. Seite 178, bekannt. Auf Seite 178 findet sich in den letzten vier Zeilen die Hydrolyse und Decarboxylierung des 2,5-Dicarboethoxy-1,4--cyclohexandions. Diese Reaktion liefert eine Ausbeute von 81 - 89%, d.h. 11 - 19% Nebenprodukte, die im rohen Reaktionsgemisch enthalten sind.

Die Umsetzung von 1,4-Cyclohexandion mit Malodinitril ist auf Seite 16 beschrieben. Es besteht nun die Befürchtung, dass Verunreinigungen des 1,4--Cyclohexandions bei dieser Reaktion mitreagieren und ein verunreinigtes 1,4-Bis-dicyanomethylencyclohexan als Endprodukt liefern. Dies ist aber für die Weiterverwendung schädlich. Überraschenderweise wurde gefunden, dass in Wirklichkeit ein äusserst reines Produkt entsteht.

Für das Verfahren geeignete Ausgangsverbindungen sind Cyclohexan-1,4-dion-2,5-dicarbonsäurealkylester der Formel (I)

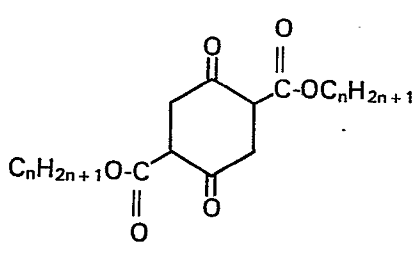

(I)

worin

n eine ganze Zahl von 1 bis 6 bedeutet.

Die oben beschriebene Umsetzung mit Wasser (Verseifung und Decarboxylierung) sowie die Kondensation mit Malonsäuredinitril können in einem Gefäss («Eintopfreaktion») in Wasser oder in einem wasserhaltigen organischen oder anorganischen Lösungsmittel durchgeführt werden.

Für die Umsetzung mit Wasser geeignete Lösungsmittel sind Alkanole wie Ethanol, Butanol; Dimethylformamid; Glykole wie Ethylenglykol und Dialkylsulfoxide, besonders bevorzugt Dimethylsulfoxid.

Die Menge an organischem oder anorganischem Lösungsmittel kann 0 bis 100, bevorzugt 0 bis 5 Gew.-Teile pro Gewichtsteil Cyclohexan-1,4-dion--2,5-dicarbonsäurealkylester sein.

Pro Mol Cyclohexan-1,4-dion-2,5-dicarbonsäurealkylester können 2 bis 200 Mol, vorzugsweise 7 bis 100 Mol, besonders bevorzugt 10 bis 15 Mol Wasser eingesetzt werden. Die Umsetzung mit Wasser wird im allgemeinen bei Temperaturen zwischen 80 und 250°C, bevorzugt 120 bis 200°C und insbesondere 120 bis 170°C, gegebenenfalls unter einem Druck von 1 bis 200 bar ausgeführt, wobei die angegebenen Werte auch darüber oder darunter liegen können.

Die Reaktionszeit kann 1 bis 24 Stunden sein, vorzugsweise 2 bis 4 Stunden.

Bei einer speziellen Ausführungsform der ersten Stufe des Verfahrens, wobei die Verseifung in wasserhaltigem Dimethylsulfoxid durchgeführt wird, kann es vorteilhaft sein, dem Reaktionsgemisch zur Erreichung kürzerer Reaktionszeiten und/oder -temperaturen 0,05 bis 2 Gew.-Teile eines Alkalihalogenids, z.B. Natriumchlorid oder Kaliumchlorid, bezogen auf den eingesetzten Cyclohexan-1,4-dion-2,5--dicarbonsäurealkylester, zuzusetzen.

Das Reaktionsgemisch der Umsetzung mit Wasser kann sofort ohne weitere Reinigung bzw. ohne Isolierung eines Zwischenproduktes mit Malonsäuredinitril zum 1,4-Bis-(dicyanomethylen)-cyclohexan umgesetzt werden.

Hierzu kann man gegebenenfalls nach Zugabe von Wasser oder einem geeigneten organischen Lösungsmittel der wässrigen Lösung 2 bis 3 Mol, vorzugsweise 2,1 bis 2,3 Mol, Malonsäuredinitril und 0 bis 0,5 Mol, vorzugsweise 0 bis 0,2 Mol, eines Katalysators, jeweils bezogen auf 1 Mol Cyclohexan--1,4-dion-dicarbonsäurealkylester, zusetzen.

Als Katalysatoren sind organische oder anorganische Basen geeignet, z.B. Alkylamine wie Diethylamin, Triethylamin; Arylamine wie Anilin, N,N-Dimethylanilin; stickstoffhaltige Heterocyclen wie Pyridin, Piperidin; Aminocarbonsäuren wie Aminoessigsäure, β-Alanin; Alkalicarbonate wie Natriumcarbonat, Kaliumcarbonat, Natriumhydrogencarbonat; Alkalihydroxide wie Natriumhydroxid, Kaliumhydroxid; Erdalkalicarbonate wie Calciumcarbonat; Erdalkalihydroxide wie Calciumhydroxid, Bariumhydroxid; Ammoniumsalze wie Ammoniumacetat, Ammoniumpropionat. Die Katalysatoren können allein oder als Gemische eingesetzt werden. Besonders bevorzugt ist eine Kombination aus 0,0005 bis 0,01 Mol β-Alanin und 0 bis 0,2 Mol Natriumhydrogencarbonat, bezogen auf eingesetzten Cyclohexan-1,4-dion-2,5-dicarbonsäurealkylester.

Im allgemeinen dauert die Umsetzung mit Malonsäuredinitril 5 bis 15 Minuten. Die Reaktionstemperatur liegt vorzugsweise zwischen 20 und 100°C.

Das 1,4-Bis-(dicyanomethylen)-cyclohexan kann durch Filtration aus der Reaktionsmischung gewonnen werden.

Die Ausbeuten liegen zwischen 90 und 96% der Theorie, bezogen auf eingesetzten Cyclohexan-1,4--dion-dicarbonsäurealkylester.

Eine spezielle Variante des Verfahrens besteht darin, dass man dem Reaktionsgemisch vor der Verseifung und Decarboxylierung Malonsäuredinitril und gegebenenfalls einen Katalysator bzw. ein Katalysatorgemisch zusetzt und so direkt das 1,4-Bis--(dicyanomethylen)-cyclohexan erhält.

Herstellung von 1,4-Bis-(dicyanomethylen)-cyclohexan.

*Beispiel 1*

114 Gew.-Teile Cyclohexan-1,4-dion-2,5-dicarbonsäuredimethylester und 200 Vol.-Teile Wasser werden in einem Rührautoklaven 4 Stunden auf 170°C erhitzt. Die abgekühlte und entspannte Lösung wird mit Wasser auf 1300 Vol.-Teile Gesamtvolumen verdünnt. Nach Zugabe von 0,1 Gew.-Teilen β-Alanin, 20 Gew.-Teilen gesättigter, wässriger Natriumhydrogencarbonatlösung und 72,6 Gew.-Teilen Malonsäuredinitril wird 5 Minuten bei 50°C gerührt. Anschliessend wird abgekühlt, der ausgefallene Feststoff abgesaugt und mit Wasser gewaschen. Es werden 94,6 Gew.-Teile (91% d. Th.) Produkt vom Schmelzbereich 204-208°C erhalten.

*Beispiel 2*

128 Gew.-Teile Cyclohexan-1,4-dion-2,5-dicarbonsäurediethylester werden, wie im Beispiel 1 beschrieben, umgesetzt. Es werden 95,7 Gew.-Teile (92% d. Th.) Produkt vom Schmelzbereich 206-209°C erhalten.

*Beispiel 3*

114 Gew.-Teile Cyclohexan-1,4-dion-2,5-dicarbonsäuredimethylester werden, wie im Beispiel 1 beschrieben, umgesetzt, mit dem Unterschied, dass nach der Verseifung nicht mit Wasser verdünnt wird. Es werden 93,6 Gew.-Teile (90% d. Th.) Produkt vom Schmelzbereich 204-206°C erhalten.

*Beispiel 4*

57 Gew.-Teile Cyclohexan-1,4-dion-2,5-dicarbonsäuredimethylester, 120 Vol.-Teile Dimethylsulfoxid, 15 Vol.-Teile Wasser und 5 Gew.-Teile Natriumchlorid werden etwa 8 Stunden unter Rückfluss (ca. 130°C) gerührt, bis die $CO_2$-Entwicklung beendet ist. Die abgekühlte Lösung wird mit 500 Vol.-Teilen Wasser verdünnt. Nach Zugabe von 0,1 Gew.-Teilen β-Alanin, 5,0 Gew.-Teilen Natriumhydrogencarbonat und 36,3 Gew.-Teilen Malonsäuredinitril wird 10 Minuten bei 50°C gerührt. Anschliessend wird mit einem Eisbad gekühlt, der Feststoff abgesaugt, mit Wasser gewaschen und getrocknet. Es werden 48,3 Gew.-Teile (93% d. Th.) Produkt von Schmelzbereich 206-210°C erhalten.

$C_{12}H_8N_4$ (208)

| | | | |
|---|---|---|---|
| Ber.: | 69,2% C | 3,8% H | 26,9% N |
| Gef.: | 69,2% C | 3,8% H | 26,9% N |

**Patentansprüche**

1. Verfahren zur Herstellung von 1,4-Bis-(dicyanomethylen)-cyclohexan, dadurch gekennzeichnet, dass man einen Cyclohexan-1,4-dion-2,5-dicarbonsäure-($C_1$-$C_6$)-alkylester mit Wasser, gegebenenfalls unter Druck von 1 bis 200 bar, bei Temperaturen von 80 bis 250°C behandelt und die erhaltene wässrige Lösung mit Malonsäuredinitril, gegebenenfalls in Gegenwart eines Katalysators, bei Temperaturen von 0 bis 100°C umsetzt.

2. Verfahren zur Herstellung von 1,4-Bis-(dicyanomethylen)-cyclohexan nach Anspruch 1, dadurch gekennzeichnet, dass man als Katalysator ein Gemisch aus β-Alanin und Natriumhydrogencarbonat verwendet.

**Claims**

1. Process for the production of 1,4-bis-(dicyanomethylene)-cyclohexane, characterised in that a cyclohexane-1,4-dione-2,5-dicarboxylic acid ($C_1$-$C_6$)-alkyl ester is treated with water, optionally under a pressure of 1 to 200 bars, at temperatures of 80 to 250°C and the aqueous solution obtained is reacted with malonic acid dinitrile, optionally in the presence of a catalyst, at temperatures of 0 to 100°C.

2. Process for the production of 1,4-bis-(dicyanomethylene)-cyclohexane according to claim 1, characterised in that a mixture of β-alanine and sodium hydrogen carbonate is used as the catalyst.

**Revendications**

1. Procédé pour la fabrication de 1,4-bis-(dicyanométhylène)-cyclohexane, caractérisé en ce que l'on traite un cyclohexane-1,4-dione-2,5-dicarboxylate d'alkyle en $C_1$-$C_6$ par l'eau, éventuellement sous une pression de 1 à 200 bars, à des températures de 80 à 250°C et l'on fait réagir la solution aqueuse obtenue avec le dinitrile malonique, éventuellement en présence d'un catalyseur, à des températures de 0 à 100°C.

2. Procédé pour la fabrication de 1,4-bis-(dicyanométhylène)-cyclohexane selon la revendication 1, caractérisé en ce que l'on utilise comme catalyseur un mélange de β-alanine et de bicarbonate de sodium.